# EUROPEAN PATENT APPLICATION

(11) **EP 3 858 975 A1**
(43) Date of publication of application: **04.08.2021**
(21) Application number: 19866734.7
(22) Date of filing: 24.09.2019
(51) Int. Cl.: C12N 1/04, A01N 1/02, A01N 63/00, C12N 5/071, C12N 5/074, C12N 5/077, C12N 5/0775

(54) **MAMMAL CELL PRESERVING SOLUTION CONTAINING ACARBOSE OR STACHYOSE**

(30) Priority: 28.09.2018 JP 2018184779
(71) Applicant: Otsuka Pharmaceutical Factory, Inc., Naruto-shi, Tokushima 772-8601 (JP)
(72) Inventor: SHIRAKAWA, Chikage, Naruto-shi, Tokushima 772-8601 (JP); NISHIMURA, Masuhiro, Naruto-shi, Tokushima 772-8601 (JP); DOI, Masako, Naruto-shi, Tokushima 772-8601 (JP)
(74) Representative: Hamer, Christopher K.
(86) International application number: PCT/JP2019/037223
(87) International publication number: WO 2020/066991

(57) **Abstract**

An object of the present invention is to provide a substance capable of effectively suppressing the decrease in cell viability and the cell aggregation that occur when mammalian cells are preserved in a liquid, and less likely to adversely affect, when administered *in vivo* to a mammal, the body of the mammal, and a mammalian cell preservation liquid containing such a substance, and the like. Use of a liquid containing acarbose or a salt thereof and/or stachyose or a salt thereof for preserving a mammalian cell can effectively suppress the decrease in cell viability and the cell aggregation that occur when mammalian cells are preserved in a liquid. Further, the mammalian cell preserved in the liquid can be directly administered *in vivo* to a mammal, without being transferred into a new transplantation liquid.

## Description

### Technical Field

The present invention relates to a liquid for preserving a mammalian cell containing acarbose or a salt thereof (which may be hereinafter referred to as "acarbose group") and/or stachyose or a salt thereof (which may be hereinafter referred to as "stachyose group") (which may be hereinafter referred to as "this mammalian cell preservation liquid "), and a method for preserving a mammalian cell using this mammalian cell preservation liquid.

### Background Art

In recent years, rapid development of stem cell research has increased the momentum for regenerative medicine, and its knowledge and understanding have become widespread not only for researchers but also for the general public. Regenerative medicine using stem cells is medical care aiming to recover the functions of cells and tissues damaged by various diseases by utilizing the self-renewal ability and pluripotency of stem cells and the factors secreted by stem cells. When bone marrow is transplanted on a patient with an intractable blood disease such as leukemia and aplastic anemia, hematopoietic stem cells engraft in the patient's body, and the hematopoietic ability can be maintained for almost a lifetime. Recently, many researchers have identified stem cells in the central nerves, peripheral nerves, bone marrow, small intestine, etc., with the aim of clinical application using stem cells other than hematopoietic stem cells, and practice of tissue stem cell transplantation therapy for traumatic diseases and tissue degenerative diseases has begun (Non-Patent documents 1 to 3). Meanwhile, cancer immunotherapy is a state-of-the-art cell therapy in which immune cells that have the function of attacking cancer are taken out of the body, the function is strengthened, and then the cells are returned to the body. Therapies using T cells, such as dendritic cell vaccine therapy, alpha-beta T cells therapy (αβ T cells therapy), gamma-delta T cells therapy (γδ T cells therapy), CTL therapy, and natural killer cell therapy (NK cell therapy) have been practiced.

When stem cells and T cells for use in transplantation therapy are preserved for a long time, the cell viability cannot be maintained well by preservation in a liquid. For example, it has been reported that, when human bone marrow stem cells are refrigerated (4°C) in saline, the cell viability decreases to 40 % or less 48 hours later and decreases to 20 % or less 72 hours later (Non-Patent document 4). Therefore, when stem cells for transplantation and T cells for transplantation are preserved for a long time, cryopreservation is common. However, there has been a problem that, since a cryopreservation agent such as DMSO and glycerol is generally added to a cryopreservation liquid, it is necessary to remove the cryopreservation agent before transplantation therapy, after thawing the stem cells and T cells cryopreserved, which is troublesome. Further, it has been also a problem that, even if a cryopreservation agent is added to a cryopreservation liquid, the cytoskeleton is significantly damaged due to the crystallization of water during freezing, and the cell viability decreases after freezing and thawing. Therefore, there has been an urgent need to develop a cell preservation liquid that is excellent in convenience and can suppress the decrease in cell viability.

The present inventors have reported that trehalose has an action of suppressing the decrease in cell viability and the cell aggregation that occur when mammalian cells are preserved in a liquid (Patent documents 1 and 2). However, it has not been known so far that acarbose and stachyose have such an action.

### Prior Art Documents

### Patent Documents

[Patent document 1] Japanese unexamined Patent Application Publication No. 2012-115253
[Patent document 2] International Publication No. WO 2014/208053

### Non Patent Documents

[Non-Patent document 1] Gage, F.H., Science 287: 1433-1438
(2000) [Non-Patent document 2] Morrison, S.J. et al., Cell 96: 737-749 (1999)
[Non-Patent document 3] Batle, E. et al., Cell 111: 251-263
(2002) [Non-Patent document 4] Lane, T.A. et al., Transfusion 49: 1471-1481 (2009)

### Summary of the Invention

### Object to be Solved by the Invention

An object of the present invention is to provide a substance capable of effectively suppressing the decrease in cell viability and the cell aggregation that occur when mammalian cells are preserved in a liquid, and less likely to adversely affect, when administered *in vivo* to a mammal, the body of the mammal, and a mammalian cell preservation liquid containing the substance, and the like.

### Means to Solve the Object

The present inventors have continued dedicated studies in order to solve the aforementioned problems. In the process, they have found acarbose and stachyose out of 93 kinds of saccharides as those that suppress the decrease in viability of mammalian cells and have a higher effect than trehalose. It was further confirmed that these tetrasaccharides have an action of suppressing aggregation of mammalian cells. The present invention has been accomplished based on these findings.

That is, the present invention is as follows.
[1] A liquid for preserving a mammalian cell comprising acarbose or a salt thereof and/or stachyose or a salt thereof.
[2] The liquid according to [1] above, wherein the acarbose or a salt thereof and/or the stachyose or a salt thereof is comprised in an isotonic liquid.
[3] The liquid according to [2] above, wherein the concentration of the acarbose or a salt thereof in the isotonic liquid is at least 40 mM.
[4] The liquid according to [2] or [3] above, wherein the concentration of the stachyose or a salt thereof in the isotonic liquid is at least 40 mM.
[5] The liquid according to any one of [2] to [4] above, wherein the isotonic liquid is a lactated Ringer's solution, an acetated Ringer's solution, a Ringer's solution, or a physiological saline.
[6] The liquid according to any one of [1] to [5] above, for preserving a mammalian cell at 0 to 40°C.
[7] The liquid according to any one of [1] to [6] above, for preserving a mammalian cell for 6 hours to 30 days.
[8] The liquid according to any one of [1] to [7] above, for use in suppressing a decrease in viability of the mammalian cell.
[9] The liquid according to any one of [1] to [8] above, for use in suppressing aggregation of the mammalian cell.
[10] The liquid according to any one of [1] to [9] above, for use in a transplantation of the mammalian cell.
[11] The liquid according to any one of [1] to [10] above, wherein the mammalian cell is a mammalian stem cell, a mammalian lymphocyte, or a mammalian hepatocyte.
[12] The liquid according to [11] above, wherein the mammalian stem cell is a mammalian mesenchymal stem cell or a mammalian lymphocyte.
[13] A method for preserving a mammalian cell, comprising a step of preserving a mammalian cell in a liquid containing acarbose or a salt thereof and/or stachyose or a salt thereof.
[14] The method according to [13] above, wherein the liquid is an isotonic liquid.
[15] The method according to [14] above, wherein the concentration of the acarbose or a salt thereof in the isotonic liquid is at least 40 mM.
[16] The method according to [14] or [15] above, wherein the concentration of the stachyose or a salt thereof in the isotonic liquid is at least 40 mM.
[17] The method according to any one of [14] to [16] above, wherein the isotonic liquid is a lactated Ringer's solution, an acetated Ringer's solution, a Ringer's solution, or a physiological saline.
[18] The method according to any one of [13] to [17] above, wherein the mammalian cell is preserved at 0 to 40°C.
[19] The method according to any one of [13] to [18] above, wherein the mammalian cell is preserved for 6 hours to 30 days.
[20] The method according to any one of [13] to [19] above, wherein the mammalian cell is a mammalian stem cell, a mammalian lymphocyte, or a mammalian hepatocyte.
[21] The method according to [20] above, wherein the mammalian stem cell is a mammalian mesenchymal stem cell or a mammalian lymphocyte.
[22] A powder formulation for preparing the liquid according to any one of [1] to [12] above, comprising acarbose or a salt thereof and/or stachyose or a salt thereof.

Examples of other embodiments of the present invention can include: a method for transplanting a mammalian cell, comprising a step of administering this mammalian cell preservation liquid containing a mammalian cell to a subject in need of transplantation of a mammalian cell (for example, a traumatic disease patient, a tissue degenerative disease patient, or a cancer patient); a method for transplanting a mammalian cell, comprising a step of preparing this mammalian cell preservation liquid by adding a mammalian cell to a liquid (preferably, isotonic liquid) containing acarbose group and/or stachyose group or adding acarbose group and/or stachyose group to a liquid (preferably, isotonic liquid) containing a mammalian cell, a step of preserving a mammalian cell in this mammalian cell preservation liquid prepared, and a step of administering this mammalian cell preservation liquid containing the preserved mammalian cell to a subject in need of transplantation of a mammalian cell (for example, a traumatic disease patient, a tissue degenerative disease patient, or a cancer patient); use of acarbose group and/or stachyose group in production of this mammalian cell preservation liquid; use of acarbose group and/or stachyose group for suppressing a decrease in viability of the mammalian cell in a liquid; use of acarbose group and/or stachyose group for suppressing aggregation of the mammalian cell in a liquid; this mammalian cell preservation liquid containing a mammalian cell, for use in treating a disease that requires mammalian cell transplantation therapy (for example, a traumatic disease, a tissue degenerative disease, or a cancer); a method for preparing this mammalian cell preservation liquid containing a mammalian cell, comprising a step of preparing this mammalian cell preservation liquid by adding a mammalian cell to a liquid containing acarbose group and/or stachyose group (preferably, isotonic liquid) or adding acarbose group and/or stachyose group to a liquid (preferably, isotonic liquid) containing a mammalian cell; and this mammalian cell preservation liquid containing mammalian cells. In the transplantation method, the step of preserving a mammalian cell is generally preserving this mammalian cell preservation liquid containing a mammalian cell under a temperature condition in which the preservation liquid is present in the form of a liquid and does not include a step of preserving the cells in the preservation liquid in the form of a solid (for example, steps of preserving a mammalian cell in a dormant state such as a cryopreservation step and a lyophilization step).

### Effect of the Invention

According to the present invention, the decrease in cell viability and the cell aggregation that occur when mammalian cells are preserved in a liquid can be effectively suppressed by adding acarbose group and/or stachyose group into the liquid. Further, these acarbose group and stachyose group are tetrasaccharides that are less likely to adversely affect, when administered *in vivo* to a mammal, the body of the mammal. Therefore, mammalian cells are preserved in this mammalian cell preservation liquid, and then can be directly administered *in vivo* to the mammal without replacing the liquid with a new transplantation liquid.

### Brief Description of Drawings

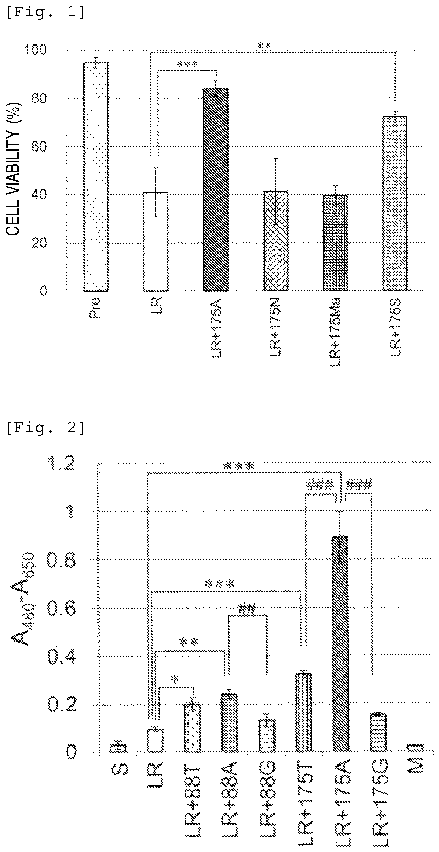

[Figure 1] Figure 1 is a graph showing the analysis results of the viability of bone marrow-derived human mesenchymal stem cells (hMSCs) after preservation in 5 kinds of test isotonic liquids (Lactec injection [LR], Lactec injection containing 175 mM acarbose [LR + 175A], Lactec injection containing 175 mM nystose [LR + 175N], Lactec injection containing 175 mM maltotetraose [LR + 175Ma], and Lactec injection containing 175 mM stachyose [LR + 175S]) at 4°C for 2 days by the trypan blue staining method (n = 3) (see Table 1). In the figure, "Pre" indicates the viability of hMSCs (94.7 ± 2.1) in phosphate buffered saline (PBS) containing hMSCs before preservation at 4°C. In the figure, "**" and "***" each indicate that there was a statistically significant difference as a result of Dunnett multiple comparison test (respectively, p < 0.01 and p < 0.001).
[Figure 2] Figure 2 is a graph showing the analysis results of the viability of hMSCs after preservation in 9 kinds of test isotonic liquids (Otsuka saline injection [S], Lactec injection [LR], Lactec injection containing 88 mM trehalose [LR + 88T], Lactec injection containing 88 mM acarbose [LR + 88A], Lactec injection containing 88 mM glucose [LR + 88G], Lactec injection containing 175 mM trehalose [LR + 175T], Lactec injection containing 175 mM acarbose [LR + 175A], Lactec injection containing 175 mM glucose [LR + 175G], and a medium [M]) at 4°C for 3 days by the XTT method (n = 3 for those other than the medium, and n = 2 for the medium) (see Table 2). In the figure, "*", "**", and "***" each indicate that there was a statistically significant difference as a result of Dunnett multiple comparison test (respectively, p < 0.05, p < 0.01 and p < 0.001). In the figure, "##" and "###" each indicate that there was a statistically significant difference as a result of Tukey type multiple comparison test among the three 88-mM groups (LR + 88T, LR + 88A, and LR + 88G) and among the three 175-mM groups (LR + 175T, LR + 175A, and LR + 175G) (respectively, p < 0.01 and p < 0.001).

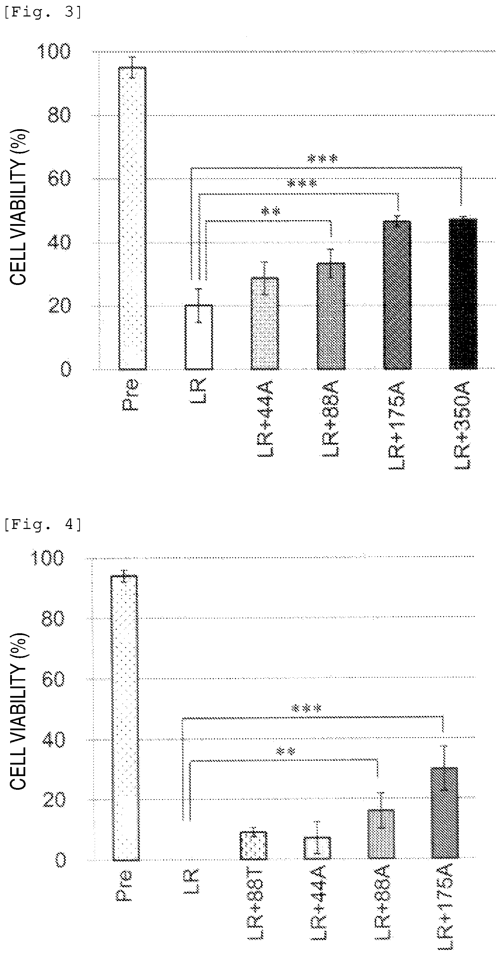

[Figure 3] Figure 3 is a graph showing the analysis results of the viability of hMSCs after preservation in 5 kinds of test isotonic liquids (Lactec injection [LR], Lactec injection containing 44 mM acarbose [LR + 44A], Lactec injection containing 88 mM acarbose [LR + 88A], Lactec injection containing 175 mM acarbose [LR + 175A], and Lactec injection containing 350 mM acarbose [LR + 350A]) at 4°C for 3 days by the trypan blue staining method (n = 3) (see Table 3). In the figure, "Pre" indicates the viability of hMSCs (95.0%) in PBS containing hMSCs before preservation at 4°C. In the figure, "**" and "***" each indicate that there was a statistically significant difference as a result of Dunnett multiple comparison test (respectively, p < 0.01 and p < 0.001).
[Figure 4] Figure 4 is a graph showing the analysis results of the viability of hMSCs after preservation in 5 kinds of test isotonic liquids (Lactec injection [LR], Lactec injection containing 88 mM trehalose [LR + 88T], Lactec injection containing 44 mM acarbose [LR + 44A], Lactec injection containing 88 mM acarbose [LR + 88A], and Lactec injection containing 175 mM acarbose [LR + 175A]) at 4°C for 28 days by the trypan blue staining method (n = 3) (see
Table 4). In the figure, "Pre" indicates the viability of hMSCs (94.1%) in PBS containing hMSCs before preservation at 4°C. In the figure, "**" and "***" each indicate that there was a statistically significant difference as a result of Dunnett multiple comparison test (respectively, p < 0.01 and p < 0.001).

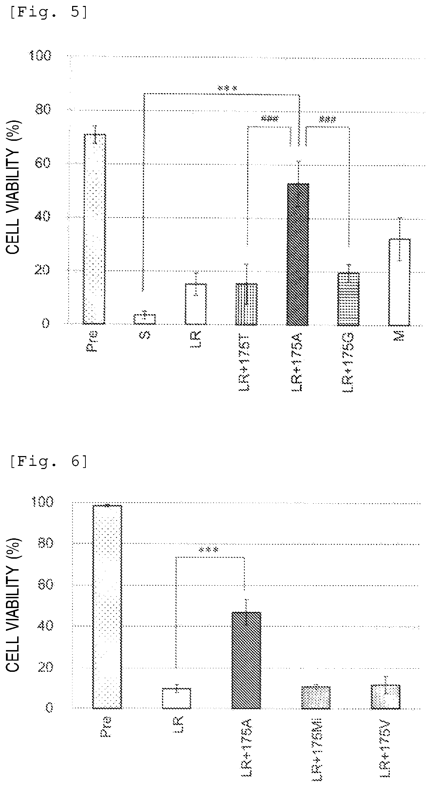

[Figure 5] Figure 5 is a graph showing the analysis results of the viability of rat hepatocytes after preservation in 6 kinds of test isotonic liquids (Otsuka saline injection [S], Lactec injection [LR], Lactec injection containing 175 mM trehalose [LR + 175T], Lactec injection containing 175 mM acarbose [LR + 175A], Lactec injection containing 175 mM glucose [LR + 175G], and a medium [M]) at 4°C for 24 hours by the trypan blue staining method (n = 6) (see Table 6). In the figure, "Pre" indicates the viability of rat hepatocytes (70.8 ± 3.32) in a wash medium containing rat hepatocytes before preservation at 4°C. In the figure, "***" indicates that there was a statistically significant difference as a result of Dunnett multiple comparison test (p < 0.001). In the figure, "###" indicates that there was a statistically significant difference as a result of Tukey type multiple comparison test among the three 175-mM groups (LR + 175T, LR + 175A, and LR + 175G) (p < 0.001).
[Figure 6] Figure 6 is a graph showing the analysis results of the viability of hMSCs after preservation in 4 kinds of test isotonic liquids (Lactec injection [LR], Lactec injection containing 175 mM acarbose [LR + 175A], Lactec injection containing 175 mM miglitol [LR + 175Mi], and Lactec injection containing 175 mM voglibose [LR + 175V]) at 4°C for 3 days by the trypan blue staining method (n = 3) (see Table 7). In the figure, "Pre" indicates the viability of hMSCs (98.5 ± 0.6) in PBS containing hMSCs before preservation at 4°C. In the figure, "***" indicates that there was a statistically significant difference as a result of Dunnett multiple comparison test (p < 0.001).

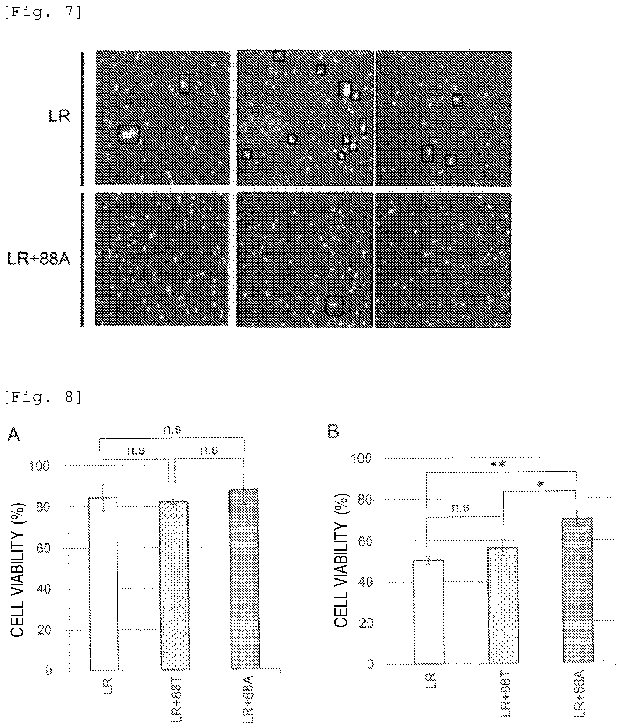

[Figure 7] Figure 7 includes phase contrast microscope images of hMSCs after preservation in two types of test isotonic liquids (Lactec injection [LR] and Lactec injection containing 88 mM acarbose [LR + 88A]) at 25°C for 48 hours. For each test isotonic liquid, a plurality of images with different fields of view were taken, and three typical images are shown. The parts circled in black in the images indicate cell aggregates.
[Figure 8] Figure 8 is a graph showing the analysis results of the viability of expansion-cultured CD8-positive T cells before preservation (Figure 8A) or after preservation (Figure 8B) in 3 kinds of test isotonic liquids (Lactec injection [LR], Lactec injection containing 88 mM trehalose [LR + 88T], and Lactec injection containing 88 mM acarbose [LR + 88A]) at 5°C for 24 hours by the trypan blue staining method (n = 3) (see Table 8) . In the figure, "*" and "**" each indicate that there was a statistically significant difference as a result of Student t test (respectively, p < 0.05 and p < 0.01) . In the figure, "n.s" indicates that there was no statistically significant difference as a result of Student t test (p ≥ 0.05).

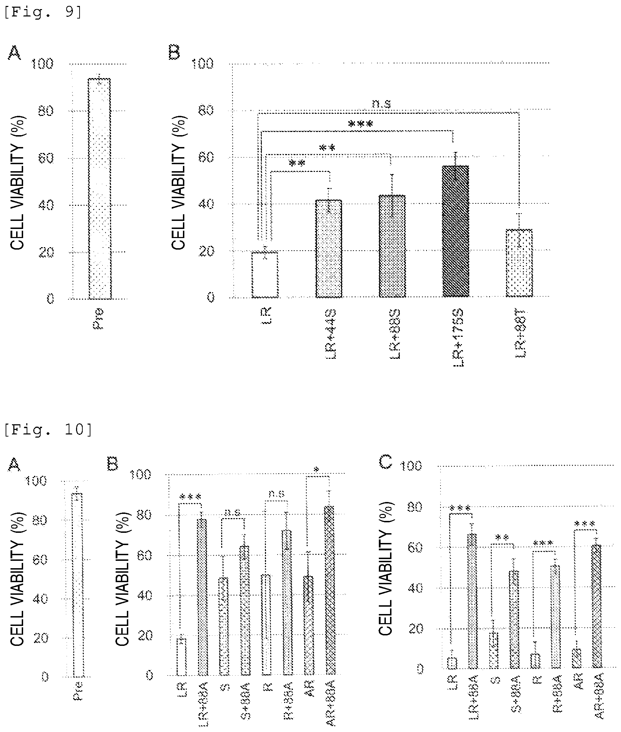

[Figure 9] Figure 9A shows the viability of hMSCs (93.7 ± 2.0%) in PBS containing hMSCs before preservation at 5°C. Figure 9B is a graph showing the analysis results of the viability of the hMSCs after preservation in 5 kinds of test isotonic liquids (Lactec injection [LR], Lactec injection containing 44 mM stachyose [LR + 44S], Lactec injection containing 88 mM stachyose [LR + 88S], Lactec injection containing 175 mM stachyose [LR + 175S], and Lactec injection containing 88 mM trehalose [LR + 88T]) at 5°C for 4 days by the trypan blue staining method (n = 3) (see Table 9). In the figure, "**" and "***" each indicate that there was a statistically significant difference as a result of Dunnett multiple comparison test (respectively, p < 0.01 and p < 0.001).
[Figure 10] Figure 10A indicates the viability of hMSCs (93.7 ± 3.2%) in PBS containing hMSCs before preservation at 5°C. Figures 10B and 10C are graphs showing the analysis results of the viability of the hMSCs after preservation in 8 kinds of test isotonic liquids (Lactec injection [LR], Lactec injection containing 88 mM acarbose [LR + 88A], Otsuka saline injection [S], Otsuka saline injection containing 88 mM acarbose [S + 88A], Ringer's solution "Otsuka" [R], Ringer's solution "Otsuka" containing 88 mM acarbose [R + 88A], Vienna F inj. [AR], and Vienna F inj. containing 88 mM acarbose [AR + 88A]) at 5°C, respectively, for 3 days and 9 days by the trypan blue staining method (n = 3) (see Table 10). In the figure, "*", "**", and "***" each indicate that there was a statistically significant difference as a result of Student t test (respectively, p < 0.05, p < 0.01, and p < 0.001). In the figure, "n.s" indicates that there was no statistically significant difference as a result of Student t test (p ≥ 0.05).

### Mode of Carrying Out the Invention

The mammalian cell preservation liquid of the present invention is a liquid (that is, this mammalian cell preservation liquid) containing acarbose group and/or stachyose group and specified for an application "for preserving a mammalian cell".

This mammalian cell preservation liquid needs only to be a liquid in which mammalian cells can be preserved (for example, an isotonic liquid, a hypotonic liquid, or a hypertonic liquid), and a preferable example thereof is an isotonic liquid. As used herein, the "isotonic liquid" refers to a liquid having an osmotic pressure substantially the same as the osmotic pressures of body fluids and cell fluids, specifically, refers to a liquid having an osmotic pressure within the range of 250 to 380 mOsm/L. As used herein, the "hypotonic liquid" refers to a liquid having an osmotic pressure lower than the osmotic pressures of body fluids and cell fluids, specifically, refers to a liquid having an osmotic pressure of less than 250 mOsm/L. The hypotonic liquid is preferably a hypotonic liquid that does not cause cells to rupture (specifically, a liquid having an osmotic pressure of less than 100 to 250 mOsm/L). As used herein, the "hypertonic liquid" refers to a liquid having an osmotic pressure higher than the osmotic pressures of body fluids and cell fluids, specifically, refers to a liquid having an osmotic pressure of over 380 mOsm/L (preferably, within the range of over 380 mOsm/L to 1000 mOsm/L) .

The isotonic liquid is not specifically limited as long as it is an isotonic liquid with a salt concentration, a sugar concentration, or the like adjusted using sodium ions, potassium ions, calcium ions, or the like so as to have substantially the same osmotic pressure as those of body fluids and cell fluids. Specifically, examples thereof can include saline, saline having a buffer effect (for example, PBS, tris buffered saline [TBS], and HEPES buffered saline), Ringer's solution, lactated Ringer's solution, acetated Ringer's solution, bicarbonated Ringer's solution, 5% glucose aqueous solution, basal media for animal cell culture (for example, DMEM, EMEM, RPMI-1640, α-MEM, F-12, F-10, and M-199), and an isotonic agent (for example, glucose, D-sorbitol, D-mannitol, lactose, and sodium chloride). Among these, lactated Ringer's solution, acetated Ringer's solution, Ringer's solution, or saline (physiological saline) is preferable. The isotonic liquid may be a commercially available product or a self-prepared product. Examples of the commercially available product can include Otsuka saline injection (manufactured by Otsuka Pharmaceutical Factory, Inc.) (physiological saline), Ringer's solution "Otsuka" (manufactured by Otsuka Pharmaceutical Factory, Inc.) (Ringer's solution), Lactec (R) injection (manufactured by Otsuka Pharmaceutical Factory, Inc.) (lactated Ringer's solution), lactated Ringer's solution "KS" (manufactured by Kyoritsu Seiyaku Corporation) (lactated Ringer's solution), Vienna F inj. (manufactured by Fuso Pharmaceutical Industries, Ltd.) (acetated Ringer's solution), 5% Otsuka sugar solution (manufactured by Otsuka Pharmaceutical Factory, Inc.) (5% glucose aqueous solution), and BICANATE injection (manufactured by Otsuka Pharmaceutical Factory, Inc.) (bicarbonated Ringer's solution).

The aforementioned acarbose is a tetrasaccharide in which D-glucose, D-glucose, D-glucose, and (1S,4R,5S,6S)-4,5,6-trihydroxy-3-(hydroxy methyl)-2-cyclohexene are linked, more specifically, a substance represented by the formula below. The acarbose can be produced by any known method such as chemical synthesis, production by microorganisms (for example, culture of amino sugar-producing bacteria, the genus Actinoplanes), and production by enzymes, but a commercially available product can also be used. Examples of the commercially available product can include acarbose (manufactured by Wako Pure Chemical Industries, Ltd.) and acarbose (product name: Glucobay) (manufactured by Bayer Yakuhin, Ltd.).

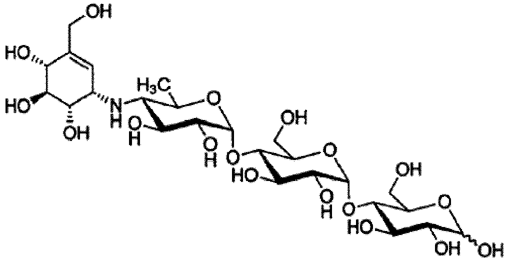

Examples of the salt of acarbose can include an acid addition salt such as hydrochloride, hydrobromide, hydroiodide, phosphate, nitrate, sulfate, acetate, propionate, toluenesulfonate, succinate, oxalate, lactate, tartrate, glycolate, methanesulfonate, butyrate, valerate, citrate, fumarate, maleate, and malate; a metal salt such as sodium salt, potassium salt, and calcium salt; an ammonium salt, and an alkyl ammonium salt. These salts are used as a solution at the time of use, and the action thereof is preferably the same as that of acarbose. These salts may form a hydrate or a solvate, and either one may be used alone, or two or more of them may be appropriately used in combination.

In the present invention, the acarbose group in the isotonic liquid may have any concentration as long as the effect of suppressing the decrease in cell viability by acarbose group is exerted and, for example, have a concentration of at least 40 mM, preferably at least 80 mM, more preferably at least 150 mM, further preferably at least 160 mM, furthermore preferably at least 170 mM. In view of the cost-effectiveness, the acarbose group in the isotonic liquid, for example, have a concentration of 1000 mM or less, preferably 700 mM or less, more preferably 600 mM or less, further preferably 500 mM or less, furthermore preferably 400 mM or less. Accordingly, the acarbose group in the isotonic liquid, for example, have a concentration within the range of 40 to 1000 mM, preferably 80 to 700 mM, more preferably 150 to 600 mM, further preferably 160 to 500 mM, furthermore preferably 170 to 400 mM.

The aforementioned stachyose is a tetrasaccharide in which D-fructose, D-glucose, D-galactose, and D-galactose are linked, more specifically, a substance represented by the formula below. The stachyose can be produced by any known method such as chemical synthesis, production by plants (for example, extraction from beans such as soybeans and Cucurbitaceae plants using hot water), and production by enzymes, but a commercially available product can also be used. Examples of the commercially available product can include stachyose n hydrate (manufactured by Wako Pure Chemical Industries, Ltd.) and stachyose hydrate (manufactured by Tokyo Chemical Industry Co., Ltd.).

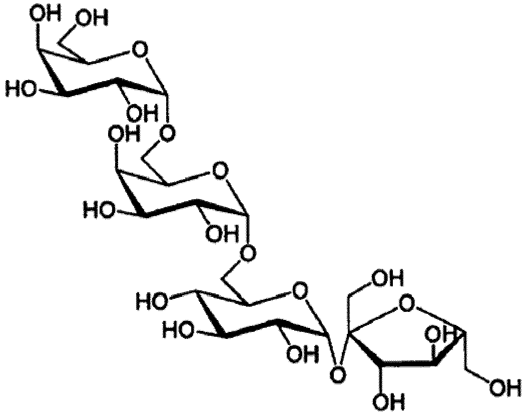

Examples of the salt of stachyose can include an acid addition salt such as hydrochloride, hydrobromide, hydroiodide, phosphate, nitrate, sulfate, acetate, propionate, toluenesulfonate, succinate, oxalate, lactate, tartrate, glycolate, methanesulfonate, butyrate, valerate, citrate, fumarate, maleate, and malate; a metal salt such as sodium salt, potassium salt, and calcium salt; an ammonium salt, and an alkyl ammonium salt. These salts are used as a solution at the time of use, and the action thereof is preferably the same as that of stachyose. These salts may form a hydrate or a solvate, and either one may be used alone, or two or more of them may be appropriately used in combination.

In the present invention, the stachyose group in the isotonic liquid may have any concentration as long as the effect of suppressing the decrease in cell viability by stachyose group is exerted and, for example, have a concentration of at least 40 mM, preferably at least 80 mM, more preferably at least 150 mM, further preferably at least 160 mM, furthermore preferably at least 170 mM. In view of the cost-effectiveness, the stachyose group in the isotonic liquid, for example, have a concentration of 1000 mM or less, preferably 700 mM or less, more preferably 600 mM or less, further preferably 500 mM or less, furthermore preferably 400 mM or less. Accordingly, the stachyose group in the isotonic liquid, for example, have a concentration within the range of 40 to 1000 mM, preferably 80 to 700 mM, more preferably 150 to 600 mM, further preferably 160 to 500 mM, furthermore preferably 170 to 400 mM.

This mammalian cell preservation liquid is used for preserving a mammalian cell for any period under a temperature condition in which this mammalian cell preservation liquid containing the mammalian cells is present in the form of a liquid. Acarbose group and stachyose group contained in this mammalian cell preservation liquid are substances having an action of effectively suppressing the decrease in cell viability and the cell aggregation that occur when the mammalian cells are preserved in a liquid and less likely to adversely affect, when administered *in vivo* to a mammal, the body of the mammal. Therefore, this mammalian cell preservation liquid is preferably further specified for one or more applications selected from: an application "for suppressing the decrease in viability of the mammalian cells"; an application "for suppressing aggregation of the mammalian cells"; and an application "for transplanting the mammalian cells".

Other than acarbose group and stachyose group, this mammalian cell preservation liquid may contain ingredients having an action of suppressing the decrease in viability of the mammalian cells. However, since acarbose group and/or stachyose group alone exert the effect of suppressing the decrease in viability of the mammalian cells, this mammalian cell preservation liquid may be free from ingredients having an action of suppressing the decrease in viability of the mammalian cells (for example, trehalose, hydroxyethyl starch [HES], and glucose) other than acarbose group and stachyose group.

This mammalian cell preservation liquid may be free from ingredients having an action of suppressing the decrease in viability of the mammalian cells generally when the mammalian cells are cryopreserved or lyophilized, such as cryoprotectants or lyoprotectants, e.g., dimethylsulfoxide [DMSO], glycerin, ethylene glycol, trimethylene glycol, dimethylacetoamide, polyethylene glycol [PEG], polyvinylpyrrolidone, serum, or serum-derived ingredients (for example, albumin).

This mammalian cell preservation liquid is preferably a liquid suitable for transplantation of mammalian cells, in the case where this mammalian cell preservation liquid containing mammalian cells is directly used for transplantation, and the liquid suitable for transplantation of mammalian cells is preferably free from substances that are not suitable for transplantation of mammalian cells, such as biological ingredients (for example, serum or serum-derived ingredients such as albumin), cryoprotectants, or lyoprotectants described above.

This mammalian cell preservation liquid may be a liquid containing acarbose group or stachyose group alone, a liquid containing two or more tetrasaccharides selected from acarbose group and stachyose group, or a liquid further containing any ingredient other than acarbose group and stachyose group.

The "any ingredient" as used herein can include an isotonic agent (for example, glucose, sorbitol, mannitol, lactose, and sodium chloride), a chelating agent (for example, EDTA, EGTA, citric acid, and salicylate), a solubilizer, a preservative, an antioxidant, an amino acid (for example, proline and glutamine), a polymer (for example, polyether), and a phospholipid (for example, lysophosphatidic acid [LPA]). As used herein, the "any ingredient" refer to an ingredient that may be contained or may not be contained.

Further, the present invention includes a powder formulation for preparing this mammalian cell preservation liquid, containing acarbose or a salt thereof and/or stachyose or a salt thereof. The powder formulation may contain any ingredient described above.

The method for preserving a mammalian cell of the present invention comprises a step of preserving a mammalian cell in a liquid containing acarbose group and/or stachyose group (that is, this mammalian cell preservation liquid) for any period (which may be hereinafter referred to as "this preservation method"). This preservation method generally stores this mammalian cell preservation liquid containing mammalian cells under a temperature condition in which the preservation liquid is present in the form of a liquid and thus does not comprise a step of preserving this mammalian cell preservation liquid under a temperature condition in which the preservation liquid is present in the form of a solid (for example, a step of preserving a mammalian cell in a dormant state, such as a cryopreservation step and a lyophilization step). Further, the density of mammalian cells in this mammalian cell preservation liquid is, for example, within the range of 10³ to 10¹⁰ cells/mL.

This preservation method may further comprise a step of preparing a liquid containing acarbose group and/or stachyose group by adding mammalian cells into a liquid (preferably, an isotonic liquid) containing acarbose group and/or stachyose group or adding acarbose group and/or stachyose group into a liquid (preferably, an isotonic liquid) containing mammalian cells, before the mammalian cells are preserved in this mammalian cell preservation liquid.

In the present invention, the period for which the mammalian cells are preserved is preferably a period that can suppress the decrease in viability of the mammalian cells in the preservation liquid and increase the percentage of living cells when this mammalian cell preservation liquid containing mammalian cells is preserved in the form of a liquid. The period is, for example, 6 hours or more, 12 hours or more, 24 hours (one day) or more, 36 hours (1.5 days) or more, 48 hours (2 days) or more, 3 days or more, 7 days or more, or 10 days or more. When the mammalian cells are preserved for an excessively long period, there may be an adverse effect on cell survival, and therefore the preservation period is generally 30 days or less, preferably 20 days or less, more preferably 10 days or less, further preferably 6 days or less, for avoiding the adverse effect on the cell viability. Accordingly, the preservation period is generally within the range of 6 hours to 30 days, within the range of 12 hours to 30 days, within the range of 24 hours to 30 days, within the range of 36 hours to 30 days, within the range of 48 hours to 30 days, within the range of 3 to 30 days, within the range of 7 to 30 days, or within the range of 10 to 30 days, preferably, 6 hours to 20 days, 12 hours to 20 days, 24 hours to 20 days, 36 hours to 20 days, 48 hours to 20 days, 3 to 20 days, 7 to 20 days, or 10 to 20 days, more preferably, 6 hours to 10 days, 12 hours to 10 days, 24 hours to 10 days, 36 hours to 10 days, 48 hours to 10 days, 3 to 10 days, or 7 to 10 days, further preferably, 6 hours to 6 days, 12 hours to 6 days, 24 hours to 6 days, 36 hours to 6 days, 48 hours to 6 days, or 3 to 6 days.

In the present invention, the "temperature at which this mammalian cell preservation liquid containing mammalian cells is present in the form of a liquid" may be a temperature at which this mammalian cell preservation liquid containing mammalian cells can be present in the form of a liquid without freezing, and the mammalian cells in the preservation liquid can grow. The temperature is generally within the range of 0 to 40°C, preferably within the range of 0 to 30°C (room temperature).

The mammalian cells of the present invention are, for example, mammalian cells to be administered via blood vessels in regenerative medicine for diseases that require mammalian cell transplantation therapy (such as a cancer; type I diabetes; and an organ disease such as liver disease), and specific examples thereof can include stem cells; pancreatic islet cells; hepatocytes; leukocytes (for example, dendritic cells, lymphocytes, macrophages, neutrophils, and eosinophils), preferably, stem cells, lymphocytes, or hepatocytes. Here, the "lymphocytes" refer to one of the subtypes of leukocytes in the immune system of mammalian cells and cells that recognize antigens and have immunity. The lymphocytes specifically include T cells (for example, alpha-beta [αβ] T cells, gamma-delta [γδ] T cells, cytotoxic T cells [cytotoxic T lymphocyte; CTL], and helper T cells), B cells, and natural killer (NK) cells.

The mammalian cells can be isolated by a known general method. For example, leukocytes and lymphocytes contained in leukocytes (for example, T cells, helper T cells, cytotoxic T cells, and γδ T cells) can be isolated from hemolyzed peripheral blood or an umbilical cord blood sample, after being expansion-cultured in the presence of IL-2 or anti-CD3 antibody, as required, by fluorescence-activated cell sorter (FACS) using an antibody against a cell surface marker of leukocytes (CD45), a cell surface marker of T cells (CD3), a cell surface marker of helper T cells (CD4), cytotoxic T cells (CD8), or a cell surface marker of γδ T cells (CD39) or by an automatic magnetic cell separator (autoMACS) using an antibody against such a cell surface marker labeled with a fluorescent substance or a labeling substance such as biotin and avidin, and a conjugate antibody of an antibody against such a labeling substance and MACS beads (magnetic beads), as cells positive for each marker. Examples of the fluorescent substance can include allophycocyanin (APC), phycoerythrin (PE), FITC (fluorescein isothiocyanate), Alexa Fluor 488, Alexa Fluor 647, Alexa Fluor 700, PE-Texas Red, PE-Cy5, and PE-Cy7.

The "stem cells" refer to immature cells having self-renewal ability and differentiation/proliferation ability. Stem cells include subpopulation such as pluripotent stem cells, multipotent stem cells, and unipotent stem cells, depending on their ability to differentiate. Pluripotent stem cells are cells that cannot become individuals by themselves but have the ability to differentiate into all tissues and cells constituting a living body. Multipotent stem cells are cells that have the ability to differentiate into multiple types of tissues and cells, though not all types. Unipotent stem cells are cells that have the ability to differentiate into specific tissues and cells.

Examples of the pluripotent stem cells can include embryonic stem cells (ES cells), EG cells (Embryonic germ cells), and induced pluripotent stem cells (iPS cells). The ES cells can be produced by culturing an inner cell mass on feeder cells or in a medium containing LIF. The method for producing ES cells is, for example, described in International Publication No. WO 96/22362, International Publication No. WO 02/101057, US Patent No. 5,843,780, US Patent No. 6,200,806, and US Patent No. 6,280,718. The EG cells can be produced by culturing primordial germ cells in a medium containing mSCF, LIF, and bFGF (Cell, 70: 841-847, 1992). The iPS cells can be produced by introducing a reprogramming factor such as Oct3/4, Sox2, and Klf4 (further c-Myc or n-Myc, as required) into somatic cells (such as fibroblasts and skin cells) (Cell, 126: p. 663-676, 2006; Nature, 448: p. 313-317, 2007; Nat Biotechnol , 26; p. 101-106, 2008; Cell 131: p. 861-872, 2007; Science, 318: p. 1917-1920, 2007; Cell Stem Cells 1: p. 55-70, 2007; Nat Biotechnol, 25: p. 1177-1181, 2007; Nature, 448: p. 318-324, 2007; Cell Stem Cells 2: p. 10-12, 2008; Nature 451: p. 141-146, 2008; and Science, 318: p. 1917-1920, 2007). Stem cells established by culturing early embryos produced by somatic cell nuclear transfer are also preferable as pluripotent stem cells (Nature, 385, 810 (1997); Science, 280, 1256 (1998); Nature Biotechnology, 17, 456 (1999); Nature, 394, 369 (1998); Nature Genetics, 22, 127 (1999); and Proc. Natl. Acad. Sci. USA, 96, 14984 (1999), and Rideout III, et.al. (Nature Genetics, 24, 109 (2000)).

Examples of the multipotent stem cells can include mesenchymal stem cells that can differentiate into cells such as adipocytes, osteocytes, chondrocytes, and adipocytes, hematopoietic stem cells that can differentiate into blood cells such as leukocytes, erythrocytes, and platelets, nervous system stem cells that can differentiate into cells such as neurons, astrocytes, and oligodendrocytes, and somatic stem cells such as bone marrow stem cells and germ stem cells. The multipotent stem cells are preferably mesenchymal stem cells. The mesenchymal stem cells are stem cells that can differentiate into all or some of osteoblasts, chondroblasts, and lipoblasts. The multipotent stem cells can be isolated from a living body by a method known per se. For example, the mesenchymal stem cells can be collected from mammalian bone marrow, adipose tissue, peripheral blood, umbilical cord blood, and the like by a known general method. For example, human mesenchymal stem cells can be isolated by culturing and passaging hematopoietic stem cells after bone marrow puncture (Journal of Autoimmunity, 30 (2008) 163-171). The multipotent stem cells can be obtained also by culturing the pluripotent stem cells under appropriate inducing conditions.

Examples of the mammals of the present invention can include a rodent such as a mouse, a rat, a hamster, and a guinea pig, *Lagomorpha* such as a rabbit, *Ungulata* such as a pig, a cow, a goat, a horse, and a sheep, *Carnivora* such as a dog and a cat, and a primate such as a human, a monkey, a rhesus monkey, a cynomolgus monkey, a marmoset, an orangutan, and a chimpanzee. Among them, a mouse, a pig, and a human are preferable.

Examples of the mammalian cells of the present invention can include adhesive (also called "adherent") cells. As used herein, the "adhesive" cells refer to scaffold-dependent cells that can survive, proliferate, and produce a substance by adhering to the scaffold. Examples of the adhesive stem cells can include pluripotent stem cells, mesenchymal stem cells, nervous system stem cells, bone marrow stem cells, and germ stem cells. Mesenchymal stem cells are preferable.

The mammalian cells (population) of the present invention may be separated from a living body or subcultured in vitro but is preferably isolated or purified. As used herein, "isolated or purified" means being subjected to an operation to remove ingredients other than the target ingredient. The purity of the mammalian cells isolated or purified (the percentage of target cells such as the number of mammalian stem cells to the total number of cells) is generally 30 % or more, preferably 50 % or more, more preferably 70 % or more, further preferably 90 % or more (for example, 100%).

The mammalian cells (population) to be preserved in this mammalian cell preservation liquid may be in the form of single cells. As used herein, the "form of single cells" means that the cells do not gather with other cells to form a mass (that is, the state where they do not aggregate). The mammalian cells in the form of single cells can be prepared by enzymatically treating mammalian cells cultured in vitro with trypsin/EDTA or the like. The percentage of the mammalian cells in the form of single cells contained in the mammalian cells is, for example, 70 % or more, preferably 90 % or more, more preferably 95 % or more, further preferably 99 % or more (for example, 100%). The percentage of the cells in the form of single cells can be determined by dispersing the mammalian cells in PBS, observing them under a microscope, and examining the presence or absence of aggregation in a plurality (for example, 1000) of cells selected at random.

The mammalian cells (population) to be preserved in this mammalian cell preservation liquid may be suspended. As used herein, "suspended" means that the mammalian cells are held in the liquid without being in contact with the inner walls of the container containing the preservation liquid.

In the case where the mammalian cells preserved in this mammalian cell preservation liquid aggregate or precipitate, the mammalian cells are preferably suspended before transplantation by a well-known method in this technical field such as pipetting and tapping.

Hereinafter, the present invention will be described more specifically by way of examples, but the technical scope of the present invention is not limited to these examples.

### Examples

### Example 1: Screening of acarbose and stachyose

The present inventors have reported so far that addition of trehalose into a liquid containing mammalian cells can suppress the decrease in cell viability when the cells are preserved in a liquid state (Japanese unexamined Patent Application Publication No. 2012-115253 and International Publication No. WO 2014/208053). Therefore, saccharides other than trehalose that exhibit the effect of suppressing the decrease in cell viability were searched for in this example.

### 1-1. Materials and methods

### [Preparation of test isotonic liquids]

93 kinds of saccharides were selected. Saccharides with clear molecular weight were each added and thawed in Lactec (R) injection that was lactated Ringer's solution (manufactured by Otsuka Pharmaceutical Factory, Inc.) to a concentration of 87.5 mM, and saccharides with unknown molecular weight were each added and thawed in Lactec injection to a concentration of 4 to 5%, to prepare isotonic liquids containing 93 kinds of saccharides. Further, trehalose (manufactured by HAYASHIBARA CO., LTD.) was added and thawed in Lactec injection to prepare an isotonic liquid containing 88 mM trehalose.

### [Mammalian cells]

The mammalian cells used were bone marrow-derived human mesenchymal stem cells (manufactured by Lonza Walkersville) (which will be hereinafter referred to as "hMSCs").

### [Preparation of PBS containing hMSCs]

PBS containing the hMSCs was prepared according to the following procedures [1] to [8].
[1] 4×10⁵ hMSCs were added to a 75-cm² flask and cultured at 37°C in a 5% CO₂ incubator in the presence of a medium. The state of the cells was observed under a microscope, and the cells were cultured until about 80% confluent.
[2] The medium was removed, and the hMSCs were rinsed with 8-mL PBS(-).
[3] The PBS(-) was removed, and 3.75-mL trypsin-EDTA (manufactured by Lonza) was added thereto, and the mixture was left standing at room temperature for 5 minutes.
[4] The hMSCs were observed under a microscope and slowly shaken until about 90% of them were exfoliated.
[5] 3.75 mL of medium was added thereto, to stop the trypsin reaction, and the hMSCs were collected by pipetting and transferred to a 50-mL centrifuge tube.
[6] Centrifugation was performed at 600× g and 22°C for 5 minutes.
[7] The supernatant (medium) was removed, 1.5 mL of PBS(-) was added thereto, and the precipitate (hMSCs) was suspended.
[8] The number of cells was measured and adjusted with PBS(-) to 5 × 10⁵ cells/mL, to prepare PBS containing hMSCs.

### [Method for preserving hMSCs in each test isotonic liquid]

The hMSCs were preserved in each test isotonic liquid according to the following procedures [1] and [2].
[1] 0.5 mL of the PBS containing hMSCs prepared was dispensed into each tube and centrifuged (at 600× g for 5 minutes).
[2] The supernatant (PBS) was removed, and the precipitate (hMSCs) was suspended in 0.5 mL of each test isotonic liquid and preserved at 4°C for 3 days.

### [Analysis of viability of hMSCs by trypan blue staining method]

The viability of hMSCs was analyzed by the trypan blue staining method according to the following procedures [1] and [2].
[1] 20 µL (equivalent to 1 × 10⁴ cells) was collected from each test isotonic liquid containing the hMSCs after preservation at 4°C for 3 days and transferred to a tube. Thereafter, 20 µL of a trypan blue staining solution (manufactured by Gibco) was mixed thereto. As a comparative control, 20 µL (equivalent to 1 × 10⁴ cells) was collected from each PBS containing hMSCs before suspension in each test isotonic liquid (before preservation at 4°C) and transferred to a tube. Thereafter, 20 µL of a trypan blue staining solution was mixed thereto.
[2] The total number of cells and the number of trypan blue-positive cells (dead cells) were measured under a microscope using OneCell Counter (manufactured by Bio Medical Science), to calculate the percentage of trypan blue-negative cells to the total number of cells, that is, the cell viability.

### 1-2. Result

Of the 93 kinds of saccharides, acarbose and stachyose were obtained as those with higher effect of suppressing the decrease in cell viability than trehalose.

Meanwhile, for D-lactobionic acid, (-)-2,3-O-iso propylidene-D-threitol, D-galacturonic acid, and D-mannosamine, the cell viability decreased as compared to the case where the hMSCs were preserved in a lactated Ringer's solution.

### Example 2: Confirmation of effect of suppressing decrease in cell viability by acarbose and stachyose

Acarbose and stachyose are tetrasaccharides in common. Therefore, in order to check whether or not tetrasaccharides other than acarbose and stachyose have an effect of suppressing the decrease in cell viability, two types of tetrasaccharides (nystose and maltotetraose) were used for examination.

### 2-1. Materials and methods

### [Preparation of test isotonic liquids]

Acarbose (manufactured by Wako Pure Chemical Industries, Ltd.) was added and thawed in Lactec injection, to prepare an isotonic liquid containing 175 mM acarbose.

Further, stachyose n hydrate (manufactured by Wako Pure Chemical Industries, Ltd.) was added and thawed in Lactec injection, to prepare an isotonic liquid containing 175 mM stachyose. Further, nystose trihydrate (manufactured by Wako Pure Chemical Industries, Ltd.) was added and thawed in Lactec injection, to prepare an isotonic liquid containing 175 mM nystose. Further, maltotetraose (manufactured by Wako Pure Chemical Industries, Ltd.) was added and thawed in Lactec injection, to prepare an isotonic liquid containing 175 mM maltotetraose. The comparative control used was Lactec injection.

### [Mammalian cells]

The mammalian cells used were the hMSCs used in Example 1.

### [Preparation of PBS containing hMSCs]

PBS containing the hMSCs was prepared according to the method described in Example 1.

### [Method for preserving hMSCs in each test isotonic liquid]

The hMSCs were preserved in each test isotonic liquid at 4°C for 2 days according to the method described in Example 1.

### [Analysis of viability of hMSCs by trypan blue staining method]

The viability of hMSCs was analyzed by the trypan blue staining method according to the method described in Example 1.

### 2-2. Result

It was confirmed that the cell viability increased when the hMSCs were preserved in Lactec injection containing acarbose or stachyose, as compared with when preserved in Lactec injection. In contrast, even when the hMSCs were preserved in Lactec injection containing nystose or maltotetraose, such an increase in cell viability was not observed (see Table 1 and Figure 1).

This result indicates that the effect of suppressing decrease in cell viability by acarbose and stachyose is not common to tetrasaccharides but is specific to acarbose and stachyose.

**[Table 1]**

| Test isotonic liquid | Cell viability (%) |
|---|---|
| | (Mean ± Standard deviation [SD]) |
| LR | 40.9±10.1 |
| LR+175A | 84.1±3.3 |
| LR+175N | 41.3±13.6 |
| LR+175Ma | 39.6±4.0 |
| LR+175S | 72.3±2.2 |

### Example 3: Confirmation of effect of suppressing decrease in cell viability by acarbose 1

The effect of suppressing the decrease in cell viability by acarbose and the effect of suppressing the decrease in cell viability by trehalose and glucose were compared and examined.

### 3-1. Materials and methods

### [Preparation of test isotonic liquids]

Acarbose (manufactured by Wako Pure Chemical Industries, Ltd.) was added and thawed in Lactec injection, to prepare an isotonic liquid containing 88 mM acarbose and an isotonic liquid containing 175 mM acarbose. Further, trehalose (manufactured by HAYASHIBARA CO., LTD.) was added and thawed in Lactec injection, to prepare an isotonic liquid containing 88 mM trehalose and an isotonic liquid containing 175 mM trehalose. Further, glucose (manufactured by Wako Pure Chemical Industries, Ltd.) was added and thawed in Lactec injection, to prepare an isotonic liquid containing 88 mM glucose and an isotonic liquid containing 175 mM glucose. The comparative controls used were Otsuka saline injection (manufactured by Otsuka Pharmaceutical Factory, Inc.) that was physiological saline, Lactec injection, and a medium kit for human mesenchymal stem cells (MSCGM BulletKit, which will be hereinafter referred to as "medium") (manufactured by Lonza Walkersville, PT-3001).

### [Mammalian cells]

The mammalian cells used were the hMSCs used in Example 1.

### [Preparation of PBS containing hMSCs]

PBS containing the hMSCs was prepared according to the method described in Example 1.

### [Method for preserving hMSCs in each test isotonic liquid]

The hMSCs were preserved in each test isotonic liquid at 4°C for 3 days according to the method described in Example 1.

### [Analysis of viability of hMSCs by XTT method]

The cell viability was analyzed by the XTT (sodium 3'-[1(-phenylaminocarbonyl)-3,4-tetrazolium]-bis(4-methoxy-6-nitro)benzene sulfonic acid hydrate) method according to the following procedures [1] to [3].
[1] 100 µL (equivalent to 5 × 10⁴ cells) was collected from each test isotonic liquid containing the hMSCs after preservation at 4°C for 3 days and transferred to a tube. Thereafter, centrifugation was performed at 600× g and 22°C for 5 minutes.
[2] The supernatant (each test isotonic liquid) was removed, 100 µL of medium was added thereto, and the precipitate (hMSCs) was suspended. Thereafter, the mixture was transferred to a 96-well plate.
[3] 50 µL of an XTT mixed reagent (mixture of an XTT reagent and an electronic coupling solution at a ratio of 50:1) of cell proliferation kit II (Cat. No. 1465015, manufactured by Roche) was added to each well.
[4] After a lapse of 24 minutes, the absorbance at 480 nm (A₄₈₀) that was an absorbance of the formazan dye metabolized and produced by living cells and the absorbance at 650 nm (A₆₅₀) that was an absorbance at the control wavelength were measured using a plate reader (Viento XS [KC junior], manufactured by Dainippon Pharmaceutical Co., Ltd.), to calculate the absorbance of A₄₈₀ corrected by A₆₅₀ (A₄₈₀-A₆₅₀) .

### 3-2. Result

When the hMSCs were preserved in Lactec injection containing 88 mM or 175 mM acarbose, the "A₄₈₀-A₆₅₀" value that was an index of living cells was higher, as compared with when preserved in Lactec injection (see Table 2 and Figure 2). Further, when the hMSCs were preserved in Lactec injection containing 88 mM or 175 mM trehalose or glucose, the "A₄₈₀-A₆₅₀" value was higher, as compared with when preserved in Lactec injection, but was significantly higher when the hMSCs were preserved in Lactec injection containing acarbose at the same concentration (see Table 2 and Figure 2) .

This result indicates that, when acarbose is added into the isotonic liquid, and the mammalian cells are preserved therein, the death of the mammalian cells in the liquid can be effectively suppressed, so that the percentage of living cells increases, as compared with the case where the mammalian cells are preserved in an isotonic liquid without acarbose or an isotonic liquid with trehalose or glucose added.

**[Table 2]**

| Test isotonic liquid | A₄₈₀-A₆₅₀ |
|---|---|
| | (Mean ± Standard deviation [SD]) |
| S | 0.028±0.017 |
| LR | 0.095±0.011 |
| LR+88T | 0.201±0.025 |
| LR+88A | 0.241±0.02 |
| LR+88G | 0.13±0.026 |
| LR+175T | 0.324±0.016 |
| LR+175A | 0.89±0.107 |
| LR+175G | 0.153±0.007 |
| M | 0.021 |

### Example 4: Examination of optimum concentration of acarbose

Using isotonic liquids containing 44 to 350 mM acarbose, the optimum concentration for the effect of suppressing the decrease in cell viability by acarbose was examined.

### 4-1. Materials and methods

### [Preparation of test isotonic liquids]

Acarbose (manufactured by Wako Pure Chemical Industries, Ltd.) was added and thawed in Lactec injection, to prepare an isotonic liquid containing 44 mM acarbose, an isotonic liquid containing 88 mM acarbose, an isotonic liquid containing 175 mM acarbose, and an isotonic liquid containing 350 mM acarbose. The comparative control used was Lactec injection.

### [Mammalian cells]

The mammalian cells used were the hMSCs used in Example 1.

### [Preparation of PBS containing hMSCs]

PBS containing the hMSCs was prepared according to the method described in Example 1.

### [Method for preserving hMSCs in each test isotonic liquid]

The hMSCs were preserved in each test isotonic liquid at 4°C for 3 days according to the method described in Example 1.

### [Analysis of viability of hMSCs by trypan blue staining method]

The viability of hMSCs was analyzed by the trypan blue staining method according to the method described in Example 1.

### 4-2. Result

When the hMSCs were preserved in Lactec injection containing 44 to 175 mM acarbose, the cell viability increased depending on the concentration of acarbose. In contrast, when the hMSCs were preserved in Lactec injection containing 175 to 350 mM acarbose, the cell viability was almost unchanged (see Table 3 and Figure 3).

This result indicates that, when acarbose of at least about 44 mM (preferably, at least about 175 mM) is added into the isotonic liquid, and the mammalian cells are preserved therein, the death of the mammalian cells in the liquid can be effectively suppressed.

**[Table 3]**

| Test isotonic liquid | Cell viability (%) |
|---|---|
| | (Mean ± Standard deviation [SD]) |
| LR | 20.1±5.2 |
| LR+44A | 28.7±5.2 |
| LR+88A | 33.3±4.5 |
| LR+175A | 46.6+1.7 |
| LR+350A | 47.1±1.0 |

### Example 5: Confirmation of effect of suppressing decrease in cell viability by acarbose when mammalian cells were preserved for long time

In order to check whether or not the effect of suppressing the decrease in cell viability by acarbose was observed also when the mammalian cells were preserved for a long time, the hMSCs were preserved in an isotonic liquid containing acarbose at 4°C for 28 days, and the cell viability was analyzed.

### 5-1. Materials and methods

### [Preparation of test isotonic liquids]

Acarbose (manufactured by Wako Pure Chemical Industries, Ltd.) was added and thawed in Lactec injection, to prepare an isotonic liquid containing 44 mM acarbose, an isotonic liquid containing 88 mM acarbose, and an isotonic liquid containing 175 mM acarbose. Further, trehalose (manufactured by HAYASHIBARA CO., LTD.) was added and thawed in Lactec injection, to prepare an isotonic liquid containing 88 mM trehalose. The comparative control used was Lactec injection.

### [Mammalian cells]

The mammalian cells used were the hMSCs used in Example 1.

### [Preparation of PBS containing hMSCs]

PBS containing the hMSCs was prepared according to the method described in Example 1.

### [Method for preserving hMSCs in each test isotonic liquid]

The hMSCs were preserved in each test isotonic liquid at 4°C for 28 days according to the method described in Example 1.

### [Analysis of viability of hMSCs by trypan blue staining method]

The viability of hMSCs was analyzed by the trypan blue staining method according to the method described in Example 1.

### 5-2. Result

When the hMSCs were preserved in Lactec injection for 28 days, the cell viability was 0%. In contrast, when the hMSCs were preserved in Lactec injection containing 88 mM acarbose for 28 days, the cell viability increased to 15.9 ± 5.90% (see Table 4 and Figure 4). Further, also when the hMSCs were preserved in Lactec injection containing trehalose for 28 days, the cell viability increased, but the percentage was higher when the hMSCs were preserved in Lactec injection containing acarbose for 28 days (see Table 4 and Figure 4). Further, it was also demonstrated that, when the hMSCs were preserved in Lactec injection containing 175 mM acarbose, the cell viability further increased, as compared with the case where the hMSCs were preserved in Lactec injection containing 88 mM acarbose (see Table 4 and Figure 4).

These results indicate that, also when acarbose is added into the isotonic liquid, and the mammalian cells are preserved therein for a long time (at 4°C for 28 days), the decrease in viability of the mammalian cells can be effectively suppressed as compared with the case where trehalose is added into the isotonic liquid and the mammalian cells are preserved for a long time.

**[Table 4]**

| Test isotonic liquid | Cell viability (%) |
|---|---|
| | (Mean ± Standard deviation [SD]) |
| LR | 0.0±0 |
| LR+88T | 8.94±1.50 |
| LR+44A | 7.00±5.27 |
| LR+88A | 15.9±5.90 |
| LR+175A | 30.0±7.38 |

### Example 6: Confirmation of effect of suppressing decrease in cell viability by acarbose 2

The effect of suppressing the decrease in cell viability by acarbose was confirmed using mammalian cells other than hMSCs (rat hepatocytes).

### 6-1. Materials and methods

### [Preparation of test isotonic liquids]

Acarbose (manufactured by Wako Pure Chemical Industries, Ltd.) was added and thawed in Lactec injection, to prepare an isotonic liquid containing 175 mM acarbose. Further, trehalose (manufactured by HAYASHIBARA CO., LTD.) was added and thawed in Lactec injection, to prepare an isotonic liquid containing 175 mM trehalose. Further, glucose (manufactured by Wako Pure Chemical Industries, Ltd.) was added and thawed in Lactec injection, to prepare an isotonic liquid containing 175 mM glucose. The comparative controls used were Otsuka saline injection, Lactec injection, and a medium.

### [Mammalian cells]

The mammalian cells used were rat hepatocytes shown in Table 5 below.

**[Table 5]**

| Mammalian cells | Rat hepatocytes |
|---|---|
| Origin | Male SD (Sprague-Dawley) rat |
| Number of cases | 13 |
| Lot number | HNA |
| Preservation method | Preserved in liquid nitrogen |
| Supply source | In Vitro Technologies |
| Agency | VERITAS Corporation |

### [Preparation of wash medium containing rat hepatocytes]

A wash medium containing rat hepatocytes was prepared according to the following procedures [1] to [6].
[1] A stock tube of rat hepatocytes was taken out of a liquid nitrogen tank and thawed in a constant temperature bath at 37°C.
[2] Immediately after thawing, it was allowed to stand on ice and transferred to a 50-mL centrifuge tube.
[3] 25 mL of hepatocyte wash medium (which will be hereinafter referred to as "wash medium") (manufactured by GIBCO) cooled with ice was added dropwise.
[4] Centrifugation was performed at 50× g and 4°C for 3 minutes.
[5] The supernatant (wash medium) was removed, 6 mL of wash medium was added thereto, and the precipitate (rat hepatocytes) was suspended by pipetting 2 to 3 times.
[6] The number of cells was measured and adjusted with a wash medium to 5 × 10⁵ cells/mL, to prepare a wash medium containing rat hepatocytes.

### [Method for preserving rat hepatocytes in each test isotonic liquid]

The rat hepatocytes were preserved in each test isotonic liquid according to the following procedures [1] and [2].
[1] 0.5 mL of the wash medium containing rat hepatocytes prepared was dispensed into each tube and centrifuged (at 50× g and 4°C for 3 minutes).
[2] The supernatant (wash medium) was removed, and the precipitate (rat hepatocytes) was suspended in 0.5 mL of each test isotonic liquid and preserved at 4°C for 24 hours.

### [Analysis of viability of rat hepatocytes by trypan blue staining method]

The viability of rat hepatocytes was analyzed by trypan blue staining method using rat hepatocytes instead of the hMSCs in the method described in Example 1.

### 6-2. Result

When rat hepatocytes were preserved in Lactec injection containing acarbose, the cell viability significantly increased, as compared with when preserved in Lactec injection or in Lactec injection containing trehalose and/or glucose (see Table 6 and Figure 5). From such a result, the effect of suppressing the cell death by acarbose is observed not only in mesenchymal stem cells (hMSCs) but also in hepatocytes and therefore is considered to be observed generally in mammalian cells.

**[Table 6]**

| Test isotonic liquid | Cell viability (%) |
|---|---|
| | (Mean ± Standard deviation [SD]) |
| S | 3.5±1.43 |
| LR | 15.0±4.14 |
| LR+175T | 15.3±7.46 |
| LR+175A | 52.9+8.62 |
| LR+175G | 19.5±3.35 |
| M | 32.5±8.07 |

### Example 7: Confirmation of effect of suppressing decrease in cell viability by acarbose 3

Acarbose binds to an active site of α-glucosidase and has an action of inhibiting hydrolysis of sugars. Therefore, in order to check whether or not the effect of suppressing the decrease in cell viability by acarbose was exerted as a result of inhibition of α-glucosidase activity in the mammalian cells, two kinds of substances (miglitol and voglibose) that were α-glucosidase inhibitors other than acarbose were used for examination.

### 7-1. Materials and methods

### [Preparation of test isotonic liquids]

Acarbose (manufactured by Wako Pure Chemical Industries, Ltd.) was added and thawed in Lactec injection, to prepare an isotonic liquid containing 175 mM acarbose. Further, miglitol (manufactured by Tokyo Chemical Industry Co., Ltd.) was added and thawed in Lactec injection, to prepare an isotonic liquid containing 175 mM miglitol. Further, voglibose (manufactured by Tokyo Chemical Industry Co., Ltd.) was added and thawed in Lactec injection, to prepare an isotonic liquid containing 175 mM voglibose. The comparative control used was Lactec injection.

### [Mammalian cells]

The mammalian cells used were the hMSCs used in Example 1.

### [Preparation of PBS containing hMSCs]

PBS containing the hMSCs was prepared according to the method described in Example 1.

### [Method for preserving hMSCs in each test isotonic liquid]

The hMSCs were preserved in each test isotonic liquid at 4°C for 3 days according to the method described in Example 1.

### [Analysis of viability of hMSCs by trypan blue staining method]

The viability of hMSCs was analyzed by the trypan blue staining method according to the method described in Example 1.

### 7-2. Result

When the hMSCs were preserved in Lactec injection containing acarbose, it was confirmed that the cell viability increased as compared with when preserved in Lactec injection. In contrast, when the hMSCs were preserved in Lactec injection containing miglitol or voglibose, the cell viability was almost unchanged, as compared with when preserved in Lactec injection (see Table 7 and Figure 6).

This result indicates that the effect of suppressing the decrease in cell viability by acarbose was not resulted from inhibition of α-glucosidase activity in the mammalian cells.

**[Table 7]**

| Test isotonic liquid | Cell viability (%) |
|---|---|
| | (Mean ± Standard deviation [SD]) |
| LR | 9.7±2.0 |
| LR+175A | 46.9±6.2 |
| LR+175Mi | 10.8±1.1 |
| LR+175V | 11.7±4.3 |

### Example 8: Effect of suppressing cell aggregation by acarbose

In order to check whether or not acarbose has an effect of suppressing cell aggregation, the hMSCs were preserved in an isotonic liquid containing acarbose, to analyze the cell aggregation level.

### 8-1. Materials and methods

### [Preparation of test isotonic liquids]

Acarbose (manufactured by Wako Pure Chemical Industries, Ltd.) was added and thawed in Lactec injection, to prepare an isotonic liquid containing 88 mM acarbose. The comparative control used was Lactec injection.

### [Preparation of PBS containing hMSCs]

PBS containing the hMSCs was prepared according to the method described in Example 1.

### [Method for preserving hMSCs in each test isotonic liquid]

The hMSCs were preserved in each test isotonic liquid at 25°C for 48 hours according to the method described in Example 1.

### [Method for analyzing cell aggregation level]

Each test isotonic liquid containing the hMSCs after preservation was partially collected, and the presence or absence of cell aggregates in which three or more cells gather together was observed under a phase contrast microscope.

### 8-2. Result

When the hMSCs were preserved in Lactec injection, a large number of cell aggregates were observed (see the parts circled in black in the upper part of Figure 7). Meanwhile, when the hMSCs were preserved in Lactec injection containing acarbose, almost no cell aggregates were observed (see the lower part of Figure 7).

This result indicates that, when acarbose is added into the isotonic liquid, and the mammalian cells are preserved therein, aggregation of the mammalian cells in the liquid can be effectively suppressed.

### Example 9: Examination of mammalian cells other than hMSCs

In order to confirm that the effect of suppressing the decrease in cell viability by acarbose was observed also when mammalian cells other than hMSCs were preserved, CD8-positive T cells, which were one of lymphocytes, were used for examination.

### 9-1. Materials and methods

### [Preparation of test isotonic liquids]

Acarbose (manufactured by Wako Pure Chemical Industries, Ltd.) was added and thawed in Lactec injection, to prepare an isotonic liquid containing 88 mM acarbose. Further, as a comparative control, trehalose (manufactured by HAYASHIBARA CO., LTD.) was added and thawed in Lactec injection, to prepare an isotonic liquid containing 88 mM trehalose. The comparative control used was Lactec injection.

### [Mammalian cells]

As the mammalian cells, CD8-positive T cells (manufactured by VERITAS) were used.

### [Method for expansion-culturing CD8-positive T cells]

CD8-positive T cells were expansion-cultured according to the following procedures [1] to [3].
[1] Cryopreserved CD8-positive T cells were thawed, then washed with a lymphocyte culture medium (LGM3, manufactured by Lonza), and incubated in the medium for about 1 hour (at 37°C in 5% CO₂) .
[2] The medium containing the CD8-positive T cells was separated and centrifuged (at 300× g and room temperature for 10 minutes).
[3] The supernatant was removed, and the cells were suspended in TLY CULTURE kit 25 (manufactured by GC LYMPHOTEC Inc.) and the CD8-positive T cells were expansion-cultured under conditions of 37°C and 5% CO₂ for 7 days.

### [Preservation of expansion-cultured CD8-positive T cells and measurement of cell viability]

Preservation of the expansion-cultured CD8-positive T cells and measurement of the cell viability were performed according to the following procedures [1] to [3].
[1] CD8-positive T cells (with a cell concentration of about 1.1 × 10⁶ cells/5 mL) expansion-cultured for 7 days were washed with Lactec injection containing 88 mM trehalose, dispensed in Stemfull tube (manufactured by Sumitomo Bakelite Co., Ltd.), and then centrifuged (at 300× g and room temperature for 10 minutes).
[2] The supernatant was removed, and the precipitate (CD8-positive T cells) was suspended in 3 kinds of test isotonic liquids (Lactec injection containing 88 mM acarbose, Lactec injection containing 88 mM trehalose, and Lactec injection) and then preserved at 5°C for 24 hours. In order to measure the cell viability before preservation, the CD8-positive T cell suspension was partially (20 µL) collected from each Stemfull tube immediately after the suspension, 20 µL of trypan blue staining solution was mixed thereto, and then the total number of cells in the area of cell-counting chambers at four corners in one cell-counting unit and the number of trypan blue-positive cells (dead cells) were measured under a microscope using OneCell Counter (manufactured by Bio Medical Science), to calculate the percentage (viability) of trypan blue-negative cells with respect to the total number of cells.
[3] After preservation for 24 hours, the cell viability was measured according to the method described in procedure [2] above.

### 9-2. Result

When the CD8-positive T cells were preserved in Lactec injection containing acarbose, the cell viability significantly increased, as compared with when preserved in Lactec injection (see Table 8 and Figure 8). Further, also when the CD8-positive T cells were preserved in Lactec injection containing trehalose, the cell viability increased, but the percentage was significantly higher when the CD8-positive T cells were preserved in Lactec injection containing acarbose (see Table 8 and Figure 8).

This result indicates that the effect of suppressing the decrease in viability by acarbose is exerted also in the case where lymphocytes such as CD8-positive T cells were preserved, and the effect is higher than that by trehalose.

**[Table 8]**

| Test isotonic liquid | Cell viability (%) | |
|---|---|---|
| | (Mean ± Standard deviation [SD]) | |
| | 0 hours | 24 hours |
| LR | 84.2±6.3 | 50.6±2.1 |
| LR+88T | 82.1±1.1 | 56.4±3.9 |
| LR+88A | 87.7±7.2 | 70.1±3.6 |

### Example 10: Examination of optimum concentration of stachyose

Using isotonic liquids containing 44 to 150 mM stachyose, the optimum concentration for the effect of suppressing the decrease in cell viability by stachyose was examined.

### 10-1. Materials and methods

### [Preparation of test isotonic liquids]

Stachyose n hydrate (manufactured by Wako Pure Chemical Industries, Ltd.) was added and thawed in Lactec injection, to prepare an isotonic liquid containing 44 mM stachyose, an isotonic liquid containing 88 mM stachyose, and an isotonic liquid containing 175 mM stachyose. Further, trehalose (manufactured by HAYASHIBARA CO., LTD.) was added and thawed in Lactec injection, to prepare an isotonic liquid containing 88 mM trehalose as a comparative control. The comparative control used was Lactec injection.

### [Mammalian cells]

The mammalian cells used were the hMSCs used in Example 1.

### [Preparation of PBS containing hMSCs]

PBS containing the hMSCs was prepared according to the method described in Example 1.

### [Method for preserving hMSCs in each test isotonic liquid]

The hMSCs were preserved in each test isotonic liquid at 5°C for 4 days according to the method described in Example 1.

### [Analysis of viability of hMSCs by trypan blue staining method]

The viability of hMSCs was analyzed by the trypan blue staining method according to the method described in Example 1.

### 10-2. Result

It was demonstrated that, when the hMSCs were preserved in Lactec injection containing stachyose, the cell viability significantly increased at any concentration of stachyose, as compared with when preserved in Lactec injection, and the cell viability increased, depending on the concentration of stachyose (see Table 9 and Figure 9). Further, the cell viability increased also when the hMSCs were preserved in Lactec injection containing trehalose, but the percentage was higher when the hMSCs were preserved in Lactec injection containing stachyose (see Table 9 and Figure 9).

This result indicates that the effect of suppressing the decrease in cell viability by stachyose is exerted when the isotonic liquid has a stachyose concentration of at least about 44 mM, and the effect was higher than that of trehalose.

**[Table 9]**

| | Cell viability (%) |
|---|---|
| Test isotonic liquid | (Mean ± Standard deviation [SD]) |
| LR | 19.3±2.6 |
| LR+44S | 41.6±5.0 |
| LR+88S | 43.5±9.0 |
| LR+175S | 55.9±5.9 |
| LR+88T | 28.5±7.1 |

### Example 11: Examination of isotonic liquids

Isotonic liquids other than Lactec injection were also examined.

### 11-1. Materials and methods

### [Preparation of test isotonic liquids]

Acarbose (manufactured by Wako Pure Chemical Industries, Ltd.) was added and thawed in each of 4 kinds of isotonic liquids (Lactec injection, Otsuka saline injection that was physiological saline [manufactured by Otsuka Pharmaceutical Factory, Inc.], Ringer's solution "Otsuka" [manufactured by Otsuka Pharmaceutical Factory, Inc.], and Vienna F inj. that was acetated Ringer's solution [manufactured by Fuso Pharmaceutical Industries, Ltd.]), to prepare the aforementioned 4 kinds of isotonic liquids containing 88 mM acarbose. Further, the aforementioned 4 kinds of isotonic liquids were used as comparative controls.

### [Mammalian cells]

The mammalian cells used were the hMSCs used in Example 1.

### [Preparation of PBS containing hMSCs]

PBS containing the hMSCs was prepared according to the method described in Example 1.

### [Method for preserving hMSCs in each test isotonic liquid]

The hMSCs were preserved in each test isotonic liquid at 5°C for 3 days (see Figure 10B) or for 9 days (see Figure 10C) according to the method described in Example 1.

### [Analysis of viability of hMSCs by trypan blue staining method]

The viability of hMSCs was analyzed by the trypan blue staining method according to the method described in Example 1.

### 11-2. Result

When the hMSCs were preserved in the 4 kinds of isotonic liquids (Lactec injection, Otsuka saline injection, Ringer's solution "Otsuka", and Vienna F inj.) containing acarbose for 3 days, the cell viability increased, as compared with when preserved in the 4 kinds of isotonic liquids for 3 days, and the cell viability particularly significantly increased when using Lactec injection and Vienna F inj. (see Table 10 and Figure 10B). Further, when the hMSCs were preserved in the 4 kinds of isotonic liquids containing acarbose for 9 days, the cell viability significantly increased, as compared with when preserved in the 4 kinds of isotonic liquids for 9 days (see Table 10 and Figure 10C).

This result indicates that the effect of suppressing the decrease in cell viability by acarbose is exerted not only in Lactec injection (that is, lactated Ringer's solution) but also generally in isotonic liquids such as Otsuka saline injection (that is, physiological saline), Ringer's solution "Otsuka" (that is, Ringer's solution), and Vienna F inj. (that is, acetated Ringer's solution).

**[Table 10]**

| Test isotonic liquid | Cell viability (%) | |
|---|---|---|
| | (Mean ± Standard deviation [SD]) | |
| | 3 days | 9 days |
| LR | 18.4±2.3 | 5.4±4.0 |
| LR+88A | 77.5±4.0 | 66.3±5.3 |
| S | 48.5±10.9 | 17.6±6.4 |
| S+88A | 64.1±5.9 | 48.1±6.2 |
| R | 49.8±31.7 | 7.1±6.1 |
| R+88A | 71.8±9.2 | 50.4±3.6 |
| AR | 48.8±12.4 | 9.3±3.9 |
| AR+88A | 83.7±7.8 | 60.5±3.5 |

### Industrial Applicability

According to the present invention, the decrease in cell viability and the cell aggregation that occur when mammalian cells are preserved in a liquid can be effectively suppressed by adding acarbose group and stachyose group into the liquid. Further, these acarbose group and stachyose group are tetrasaccharides that are less likely to adversely affect, when administered *in vivo* to a mammal, the body of the mammal. Therefore, mammalian cells are preserved in this mammalian cell preservation liquid, and then can be directly administered *in vivo* to the mammal without replacing the liquid with a new transplantation liquid.

## Claims

1. A liquid for preserving a mammalian cell comprising acarbose or a salt thereof and/or stachyose or a salt thereof.

2. The liquid according to claim 1, wherein the acarbose or a salt thereof and/or the stachyose or a salt thereof is comprised in an isotonic liquid.

3. The liquid according to claim 2, wherein the concentration of the acarbose or a salt thereof in the isotonic liquid is at least 40 mM.

4. The liquid according to claim 2 or 3, wherein the concentration of the stachyose or a salt thereof in the isotonic liquid is at least 40 mM.

5. The liquid according to any one of claims 2 to 4, wherein the isotonic liquid is a lactated Ringer's solution, an acetated Ringer's solution, a Ringer's solution, or a physiological saline.

6. The liquid according to any one of claims 1 to 5, for preserving a mammalian cell at 0 to 40°C.

7. The liquid according to any one of claims 1 to 6, for preserving a mammalian cell for 6 hours to 30 days.

8. The liquid according to any one of claims 1 to 7, for use in suppressing a decrease in viability of the mammalian cell.

9. The liquid according to any one of claims 1 to 8, for use in suppressing aggregation of the mammalian cell.

10. The liquid according to any one of claims 1 to 9, for use in a transplantation of the mammalian cell.

11. The liquid according to any one of claims 1 to 10, wherein the mammalian cell is a mammalian stem cell, a mammalian lymphocyte, or a mammalian hepatocyte.

12. The liquid according to claim 11, wherein the mammalian stem cell is a mammalian mesenchymal stem cell or a mammalian lymphocyte.

13. A method for preserving a mammalian cell, comprising a step of preserving the mammalian cell in a liquid containing acarbose or a salt thereof and/or stachyose or a salt thereof.

14. The method according to claim 13, wherein the liquid is an isotonic liquid.

15. The method according to claim 14, wherein the concentration of the acarbose or a salt thereof in the isotonic liquid is at least 40 mM.

16. The method according to claim 14 or 15, wherein the concentration of the stachyose or a salt thereof in the isotonic liquid is at least 40 mM.

17. The method according to any one of claims 14 to 16, wherein the isotonic liquid is a lactated Ringer's solution, an acetated Ringer's solution, a Ringer's solution, or a physiological saline.

18. The method according to any one of claims 13 to 17, wherein the mammalian cell is preserved at 0 to 40°C.

19. The method according to any one of claims 13 to 18, wherein the mammalian cell is preserved for 6 hours to 30 days.

20. The method according to any one of claims 13 to 19, wherein the mammalian cell is a mammalian stem cell, a mammalian lymphocyte, or a mammalian hepatocyte.

21. The method according to claim 20, wherein the mammalian stem cell is a mammalian mesenchymal stem cell or mammalian lymphocyte.

22. A powder formulation for preparing the liquid according to any one of claims 1 to 12, comprising acarbose or a salt thereof and/or stachyose or a salt thereof.
